# EUROPEAN PATENT SPECIFICATION

(11) **EP 1 631 555 B1**
(45) Date of publication and mention of the grant of the patent: **29.04.2009**
(21) Application number: 04748631.1
(22) Date of filing: 03.06.2004
(51) Int. Cl.: C07D 251/56, B01J 2/22

(54) **Process for preparing granular melamine**
Verfahren zur Herstellung von Melamingranulat
Procédé de préparation de melamine granulaire

(30) Priority: 06.06.2003 NL 1023617
(43) Date of publication of application: 08.03.2006
(73) Proprietor: DSM IP Assets B.V., 6411 TE Heerlen (NL)
(72) Inventor: VAN DIJK, Saskia, Ingeborg, NL-6215 JC Maastricht (NL); VONK, Pieter, NL-6135 JD Sittard (NL)
(74) Representative: Koster, Nico
(86) International application number: PCT/NL2004/000399
(87) International publication number: WO 2004/108693

(56) References cited:
- PATENT ABSTRACTS OF JAPAN vol. 0142, no. 54 (C-0724), 31 May 1990 (1990-05-31) & JP 02 073073 A (NISSAN CHEM IND LTD), 13 March 1990 (1990-03-13) -& DATABASE WPI Section Ch, Week 199016 Derwent Publications Ltd., London, GB; Class A41, AN 1990-121130 XP002305072 -& JP 02 073073 A (NISSAN CHEM IND LTD) 13 March 1990 (1990-03-13)

## Description

### The invention relates to a process for compacting melamine and to obtainable by said process a granular melamine.

Granular melamine is known from RD 42466. In RD 42466 the preparation of granular melamine is disclosed in general terms, via a granulation process, a compacting process or an extrusion process.

A drawback of the known granular melamine is that it typically dissolves much more slowly in solvents such as water or an aqueous solution of formaldehyde (also known as formalin) than the starting material.

The objective of the invention is to reduce the aforementioned drawback.

The objective of the invention is achieved in that the D₉₉ of the granular melamine lies between 300µm and 1800µm and multicrystalline melamine is used as start material for the compacting process.

The advantage of the granular melamine according to invention is that the rate of dissolution in solvents such as formalin is higher than that of the known granular melamine.

The melamine according to the invention is granular. This means that the melamine, in comparison with melamine obtained directly from a process for the preparation of melamine, has undergone an agglomeration step. Within the context of the present invention, a process for the preparation of melamine is understood to be any process wherein melamine is synthesized from raw materials, in particular urea, and obtained in powder form. Such processes are known per se, as disclosed in for example section 4 of the chapter 'Melamine and Guanamines' in Ullmann's Encyclopedia of Industrial Chemistry, Sixth Edition, 2001. The said known processes for the preparation of melamine do not comprise agglomeration steps within the meaning of the present invention such as granulation, compacting or extrusion as disclosed in RD 42466. In effect, the melamine powder as obtainable from the said processes constitutes a preferred starting material for the preparation of the granular material according to the invention.

The granular melamine according to the invention has a D₉₉ lying between 300 µm and 1800 µm. As is known, a 'D_{α} of β µm' means that when a particle size distribution measurement is done, the value of β µm is only reached after α wt.% of the particles have been taken into account as undersize fraction. Thus, α wt.% of the particles have a particle size of not more than β µm.

The D₉₉ of the granular melamine according to the invention should be at least 300 µm. It was found that with increasing D₉₉, the handling characteristics of the granular melamine such as its free-flowing behaviour improve. Preferably, the D₉₉ of the granular melamine according to the invention is at least 400 µm, more preferably at least 500 µm, in particular at least 600 µm and most preferably at least 700 µm.

The D₉₉ of the granular melamine according to the invention should be at most 1800 µm. It was found that the rate of dissolution of the granular melamine in solvents such as water or formalin increases if the D₉₉ is at or below 1800 µm, compared to granular melamine having a higher D₉₉ such as for example 2500 µm or more. Preferably, the D₉₉ of the granular melamine according to the invention is at most 1700 µm, more preferably at most 1600 µm, in particular at most 1500 µm and most preferably at most 1400 µm.

In a preferred embodiment of the granular melamine according to the invention, the D₅ of the granular melamine is at least 25 µm. This means that the granular melamine comprises a low percentage of very fine particles ('fines'). The advantage of this is that the flow characteristics of the granular melamine during transport and handling improve. Preferably, the D₆ of the granular melamine is at least 50 or 75 µm, more preferably at least 100 or 150 µm, in particular at least 200 µm or 250 µm, most preferably at least 300 µm. The D₅ of the granular melamine according to the invention should preferably be at most 1700 µm or 1500 µm, since an even higher value of D₅ will make it virtually impossible to achieve a D₉₉ within the range of the -present invention. More preferably, the D₅ is at most 1000 µm or 800 µm, most preferably at most 500 µm.

The granular melamine according to the invention, is obtainable by an agglomeration step done on multicrystalline melamine; more preferably, the said agglomeration step is a compacting process, It was found, surprisingly, that compacted multicrystalline melamine according to the invention has a higher rate of dissolution than the starting material, i.e. multicrystalline melamine itself. Multicrystalline melamine and its preparation are known per se, and disclosed in for example US 4,565,867 or WO 99/46251.

The compacting of melamine is disclosed in a general fashion in RD 42466. In order to arrive at the compacted granular melamine according to the invention, it must be ensured that the D₉₉ - and, if applicable, also the D₅- as given above are achieved. The invention thereto also relates to a process for compacting melamine comprising:
■ a roll pressing step wherein a multicrystalline melamine powder is pressed into compacted melamine;
■ a crushing step wherein the compacted melamine is crushed into granular melamine;
■ a coarse screening step to which the granular melamine is supplied, wherein particles greater than a desired particle size are separated from the granular melamine,
wherein the coarse screening step is carried out in such a fashion that the granular melamine has a D₉₉ lying between 300 µm and 1800 µm.

In a general fashion, a roll pressing step, crushing step and coarse screening step as such are known from RD 42466, or from Perry Chemical Engineers Handbook (6th Ed., ISBN 0-07-049479-7 McGraw-Hill Book Company, pages 8-62 through 8-65).

In the roll pressing step of the invention compacted melamine is formed. As is known, compaction means agglomeration with exertion of pressure. The roll pressing step in the process according to the invention can be effected in various ways, for example with the aid of two rotating rolls; the rolls may be smooth so that the compacted melamine emerges from the roll pressing step in the form of a plate; the rolls may also be profiled so that the compacted melamine has a pre-defined shape such as for example a cylinder. In a preferred embodiment of the roll pressing step, one roll is smooth and un-profiled, whereas the other roll is profiled. A significant operational parameter for performing the roll pressing step is the roll loading; as defined in for example Perry Chemical Engineers Handbook (6th Ed., ISBN 0-07-049479-7 McGraw-Hill Book Company, pages 8-62 through 8-65), this is the force applied in the roll pressing step divided by the roll width. Roll loading is normally expressed in Meganewton per meter of roll width (MN/m). The roll loading may vary between wide limits; however, the roll loading must not be too low, otherwise compacting will not be effective. The roll loading must not be too high, either, because otherwise the compacted melamine will be so "solid" that downstream operations such as dissolving the melamine in for example water or formalin will not proceed well. The roll pressing step according to the invention is preferably carried out with a roll loading of at least 0.3 or 0.5 MN/m, more preferably 0.75 or 1 MN/m, still more preferably at least 1.5 MN/m, especially preferably at least 2 MN/m and most preferably at least 2.5 MN/m. The roll pressing step according to the invention is preferably carried out with a roll loading of at most 8 or 7 MN/m, more preferably at most 6 or 5 MN/m, still more preferably at most 4 MN/m, especially preferably at most 3.5 MN/m and most preferably at most 3 MN/m.

The melamine powder as supplied to the roll pressing step may be any known multicrystalline melamine powder. In a preferred embodiment the melamine powder is obtained in a high-pressure - that is, at a pressure between 5 MPa and 30 MPa - non-catalytic process for the preparation of melamine. In such a high-pressure process, the melamine is obtained from a melamine melt in the form of, for example, multicrystalline material directly via an expansion step, with or without cooling with the aid of for example NH₃, or in the form of a powder essentially consisting of single crystals obtained via an aqueous recovery section such as through (re)crystallization from an aqueous phase that may or may not contain NH₃ and/or another alkaline compound such as NaOH. In another preferred embodiment the melamine powder is obtained in a low-pressure - that is, at a pressure between atmospheric and about 3 MPa - catalytic process for the preparation of melamine;-such-processes may comprise an aqueous recovery section. It is also possible for the melamine powder first to be subjected to one or more operations, such as an agglomeration step, before it is supplied to the roll pressing step.

The crushing step, wherein the compacted material, which is available in the form of, for example, a plate or (semi-)cylinder, is reduced, may be carried out in any suitable manner known per se. Granular melamine already develops in this process; however, this granular melamine may still contain particles that are larger or smaller than desired. Therefore, in the process according to the invention, the crushing step is preferably followed by a coarse screening step. The coarse screening step, which is applied to granular melamine obtained in the crushing step, may be implemented though techniques known per se, with the object of separating granular melamine that is larger than a desired size; these are coarse particles. The maximum desired size is dependent on the later requirements that are set for the granular melamine, and thus may vary between wide limits. In order to obtain the granular melamine according to the invention, the coarse screening step should be carried out in such a fashion that the granular melamine has a D₉₉ lying between 300 µm and 1800 µm. As is known to the skilled person, this can be achieved by for example selecting the size of the holes in the screen that is used in the coarse screening step such that the abovementioned D₉₉ is achieved.

The separated coarse particles are preferably returned to the crushing step. This has the advantage, in comparison with known processes wherein the coarse particles are returned to the roll pressing step, that the granular melamine has a structure such that it dissolves more rapidly than the granular melamine made by the known process. It may be desirable not to return all separated coarse particles to the crushing step; in a preferred embodiment of the process according to the invention a proportion of the separated coarse particles is added to the granular melamine all the same. The advantage of this process is that it is possible to accurately control the proportion of such coarse particles. Preferably, at most 60 wt.% of the coarse particles is added to the granular melamine; more preferably at most 50 wt.%, still more preferably at most 40 wt.% or at most 30 wt.%, especially preferably at most 20 Wt.% and most preferably at most 10 wt.% - while ensuring that the D₉₉ of the resulting granular melamine is within the limits as given above.

In a preferred embodiment, the process according to the invention also comprises a fine screening step to which the granular melamine from the coarse screening step or the crushing step is supplied, wherein particles below a desired particle size are separated from the granular melamine, whereafter the separated particles are partially returned to the roll pressing step and partially supplied to the granular melamine from the fine screening step. The fine screening step may be implemented through techniques known per se; the object of the fine screening step is to separate granular melamine that is smaller than a desired size; these are fine particles or fines. The minimum desired size of the granular melamine is dependent on the eventual application of the granular melamine, and thus may vary between wide limits. Preferably, the minimum desired size of the granular melamine is 10 µm or 20 µm, more preferably 40 µm or 60 µm, still more preferably 80 or 100 µm, especially preferably 150-or 200 µm and most preferably 250 or 300µm. As a result of the fine screening step, it becomes possible to steer the D₅ of the granular melamine so as to obtain a granular melamine according to a preferred embodiment having a D₅ of at least 200 µm. It may be advantageous to add a proportion of the separated fine particles to the granular melamine; preferably, at most 60 wt.% of the fine particles is supplied to the granular melamine; more preferably at most 50 wt.%, still more preferably at most 40 wt.% or at most 30 wt.%, especially preferably at most 20 wt.% and most preferably at most 10 wt.% - while ensuring that the D₉₉, and if applicable the D₅, of the resulting granular melamine is within the limits as given above. In a preferred embodiment of the process according to the invention the coarse screening step and fine screening step are carried out simultaneously.

It is possible in the process according to the invention to add a binder, such as for example water, to the melamine powder. In a further preferred embodiment of the process according to the invention, however, the melamine powder is supplied to the roll pressing step without a binder. Within the context of the present invention, the term 'without (the use of) a binder' means that less than 1 wt%, preferably less than 0.4 wt.%, in particular less than 0.4 wt.% and most preferably 0 wt.% of a binder is used. The weight percentages of binder are percentages of the amount of melamine as supplied to the agglomeration step. This has the advantage that it is no longer necessary for the binder to be removed and/or that any drawbacks in the further processing of the binder-containing melamine are avoided, while coherent granular melamine is nevertheless obtained.

It is possible in the process according to the invention to execute a size reduction step on the melamine powder prior to the compacting process. An example of such a size reduction process is a milling process. In a preferred embodiment, however, such a size reduction step is not done.

The process according to the invention can be carried out as a stand-alone process; alternatively, however, the process may be incorporated in a process for the synthesis of melamine.

The granular melamine according to the invention is, due to its high rate of dissolution in solvents such as water or formalin, exceptionally suitable for use in aminoplast resins. Examples of such resins are a melamine-formaldehyde resin and a melamine-urea-formaldehyde resin.

The granular melamine according to the invention has undergone an agglomeration step. As a result of the agglomeration step, the surface characteristics of the granular melamine have undergone a change as compared to the starting material. One important parameter in the surface characteristics of a material is the disperse energy content. As is known, the disperse energy content of a material one of the two components that make up the surface energy of the material, i.e. the component that is based on the Van der Waals interactions. The other component in the surface energy of a material. is formed by the polar interactions of the material. A higher disperse energy content of a material is discernible in a plurality of related material properties such as for example a higher possible static charge, less easy moistening by hydrophobic materials or increased tendency to agglomerate. A technique known per se for determining the disperse energy content of a material is Inversed Gas Chromatography (IGC). In this technique a column is filled with the material whose disperse energy content is to be determined. Subsequently, various gases having different polar characters are passed along the material and the retention time is determined for each gas. The disperse energy content can then be calculated herefrom. Measurement of the disperse energy content by IGC is described in for example 'An exploration of inter-relationships between contact angle, inverse phase gas chromatography and triboelectric charging data', N.M. Ahfat, G. Buckton, R. Burrows, M.D. Ticehurst, Eur. J. Pharm. Sci., 9 (2000), 221 - 276. It should be noted here that the disperse energy content of a material is a characteristic that is related to the surface of its particles; this means that the measurement may be affected when a material has been in contact with other substances. Preferably, therefore, the disperse energy content of a material - such as granular melamine - is measured shortly after its preparation or without the material having been influenced in any significant way, which may be assumed when a material is directly taken from its original packaging for measurement.

Surprisingly, it has been found that if the disperse energy content of granular melamine according to the invention is between 40 and 62 mJ/m², the granular melamine dissolves more rapidly in solvents such as water or formalin than known melamine, in particular non-granular melamine such as melamine directly obtained from a process for the preparation of melamine such as multicrystalline melamine; this is in spite of the granular melamine according to the invention having undergone an agglomeration step so that one..skilled in the art would expect the dissolution of the granular melamine to proceed more slowly. Preferably, the disperse energy content of the granular melamine according to the invention is at least 42 or 44 mJ/m², more preferably at least 46 or 47 mJ/m², still more preferably at least 49 or 50 mJ/m², especially preferably 51 or 52 mJ/m² and most preferably at least 53 mJ/m². Preferably, the disperse energy content of the granular melamine according to the invention is at most 66 mJ/m², more preferably at most 65 mJ/m², still more preferably at most 64 mJ/m², especially preferably at most 63 mJ/m² and most preferably at most 62 mJ/m².

The invention will be elucidated by means of Examples and Comparative Experiments.

### Example 1

Granular melamine according to the invention was prepared by a process according to the invention.

The roll pressing step was applied to a multicrystalline melamine (supplier: MCI). The roll pressing step was effected with a Bepex L200/50 P; one roll was smooth, the other roll had profile; this resulted in compacted melamine in semicircular form. The roll loading was 0.9 MN/m. After the compacted melamine was crushed into granular melamine, the coarse screening step was carried out, with all particles of 1 mm or greater being separated. The granular melamine obtained had a D₉₉ of 1000 µm.

### Example 2

The dissolution behaviour of granular melamine according to the invention prepared as in Example 1, having a D₉₉ of 1000µm, was tested in a melamine-formaldehyde resin preparation process. Resin preparation took place in a Mettler Toledo Labmax^{™} Automatic Lab Reactor, controlled by Camille TG^{™} software. Formalin with a formaldehyde content of 31 wt.% (< 200ppm formic acid, 1 % methanol) was charged into the reactor and then water was added as necessary for the solids content, being the percent sum of the weight fractions of formaldehyde and the amino compounds to be added later, relative to the total amount of resin, to be 55 wt.% . The content was heated to 30°C in 10 minutes. Next the pH was adjusted to 9.3 with the aid of a 2N NaOH solution. Subsequently, melamine was added for a Formaldehyde/Melamine molar ratio of 1.5. Then the reactor content was heated to 100°C in 47 minutes whereupon the reactor content was kept at 100°C until the cloud point was reached. The reactor content was then cooled to 95°C and kept at 95°C until, after cooling of the reactor content, a resin with a water tolerance of between 1.5 and 2.5 (20 °C) was achieved.

As is known, the cloud point is defined as the degree of reaction (degree of condensation) at which the resin is no longer infinitely soluble in water. The cloud point is normally determined by determining the point where 1 drop of resin, when added to a large amount of water at 20°C, ceases to dissolve but produces turbidity. As is known, water tolerance is defined as the amount of water (in grams) that can be added to 1 gram of resin at 20°C before the water resin mixture becomes. turbid.

The resin turned clear after 64 minutes, indicating that all melamine had dissolved; this was before the cloud point was reached, which happened after 70 minutes. A water tolerance of 2.0 was reached after 83 minutes.

### Examples 3, 4

Example 2 was repeated with two other granular melamines, prepared in analogy to example 1, also having a D₉₉ of 1000 µm.

The resins turned clear after 63 and 59 minutes, indicating that all melamine had dissolved; this was before the cloud point was reached, which happened after 71 and 69 minutes. A water tolerance of 2.0 was reached after 94 and 87 minutes.

### Comparative Experiment 1

An agglomerated, multicrystalline melamine in granular form, having a D₉₉ of 2000 µm was used in a melamine-formaldehyde resin preparation process. The resin preparation process was carried out in the same fashion as in Example 2. The resin turned clear after 75 minutes, indicating that all melamine had dissolved; this was after the cloud point was reached, which happened after 71 minutes. A water tolerance of 2.0 was reached after 83 minutes.

### Comparative Experiment 2

A non-agglomerated multicrystalline melamine in powder form, having a D₉₉ of 145 µm was used in a melamine-formaldehyde resin preparation process. The resin preparation process was carried out in the same fashion as in Example 2. The resin turned clear after 68 minutes, indicating that all melamine had dissolved; this was before the cloud point was reached, which happened after 72 minutes. A water tolerance of 2.0 was reached after 92 minutes.

From the Examples and Comparative Experiments, it clearly follows that a compacted multicrystalline melamine having a D₉₉ according to the invention has a higher rate of dissolution - shorter time until the resin turned clear - than a compacted multicrystalline melamine having a D₉₉ outside the range according to the invention. It also follows, surprisingly, that the said granular melamine according to the invention has a higher rate of dissolution than the starting material.

## Claims

1. Process for compacting melamine comprising:
■ a roll pressing step wherein a multicrystalline melamine powder is pressed into compacted melamine;
■ a crushing step wherein the compacted melamine is crushed into granular melamine;
■ a coarse screening step to which the granular melamine is supplied, wherein particles greater than a desired particle size are separated from the granular melamine,
**characterised in that** the coarse screening step is carried out in such a fashion that the granular melamine has a D₉₉ lying between 300µm and 1800µm.

2. Process according to claim 1, wherein the particles as separated in the coarse screening step from the granular melamine are returned at least partially to the crushing step and optionally at most partially supplied to the granular melamine from the coarse screening step.

3. Process according to claim 1 or 2, further comprising a fine screening step to which the granular melamine from the coarse screening step is supplied, wherein particles below a desired particle size are separated from the granular melamine, where after the separated particles are partially returned to the roll pressing step and partially supplied to the granular melamine from the fine screening step.

4. Process according to claim 3, wherein the roll loading lies between 0.3 and 7 MN/m.

5. Granular melamine obtainable by a process according to any one of claims 1-4, **characterized in that** the D₉₉ lies between 300 µm and 1800 µm.

6. Granular melamine according to claim 5, wherein the D₆ is at least 26 µm.

7. Granular melamine according to claims 5 or 6. wherein the granular melamine is obtained without the use of a binder.

8. Granular melamine according to any one of claims 5-7, wherein the granular melamine is obtained without a size reduction step prior to agglomeration.

9. Use of granular melamine according to any one of claims 5-8 in the preparation of an aminoplast resin.

## Patentansprüche

1. Verfahren zum Kompaktieren von Melamin mit:
■ einem Walzenpreßschritt, bei dem ein multikristallines Melaminpulver zu kompaktiertem Melamin gepreßt wird;
■ einem zerkleinerungsschritt, bei dem das kompaktierte Melamin zu Melamingranulat zerkleinert wird;
■ einem Grobsiebungsschritt, dem das Melamingranulat zugeführt wird, bei dem Teilchen, die größer als eine gewünschte Teilchengröße sind, von dem Melamingranulat abgetrennt werden,
**dadurch gekennzeichnet, daß** der Grobsiebungsschritt so durchgeführt wird, daß das Melamingranulat einen D₉₉-Wert zwischen 300 µm und 1800 µm aufweist.

2. Verfahren nach Anspruch 1, bei dem die im Grobsiebungsschritt von dem Melamingranulat abgetrennten Teilchen zumindest zum Teil zum zerkleinerungsschritt zurückgeführt und gegebenenfalls höchstens zum Teil dem Melamingranulat aus dem Grobsiebungsschritt zugeführt werden.

3. Verfahren nach Anspruch 1 oder 2, ferner mit einem Feinsiebungsschritt, dem das Melamingranulat aus dem Grobsiebungsschritt zugeführt wird, bei dem Teilchen, die kleiner als eine gewünschte Teilchengröße sind, von dem Melamingranulat abgetrennt werden, wonach die abgetrennten Teilchen zum Teil zum Walzenpreßschritt zurückgeführt und zum Teil dem Melamingranulat aus dem Feinsiebungsschritt zugeführt werden.

4. Verfahren nach Anspruch 3, bei dem die Walzenbelastung zwischen 0,3 und 7 MN/m liegt.

5. Melamingranulat, das nach einem Verfahren nach einem der Ansprüche 1-4 erhältlich ist, **dadurch gekennzeichnet, daß** der D₉₉-Wert zwischen 300 µm und 1800 µm liegt.

6. Melamingranulat nach Anspruch 5, bei dem der D₅-Wert mindestens 25 µm beträgt.

7. Melamingranulat nach Anspruch 5 oder 6, bei dem das Melamingranulat ohne Verwendung eines Bindemittels erhalten worden ist.

8. Melamingranulat nach einem der Ansprüche 5-7, bei dem das Melamingranulat ohne einen Größenverringerungsschritt vor der Agglomeration erhalten worden ist.

9. Verwendung von Melamingranulat nach einem der Ansprüche 5-8 bei der Herstellung eines Aminoplastharzes.

## Revendications

1. Procédé de compactage de mélamine, comprenant :
• une étape de pressage au rouleau dans laquelle une poudre de mélamine multicristalline est pressée jusqu'à former une mélamine compactée ;
• une étape de broyage dans laquelle la mélamine compactée est broyée pour former une mélamine granulaire ;
• une étape de tamisage grossier à laquelle est transférée la mélamine granulaire, dans laquelle les particules ayant une taille supérieure à une taille de particule souhaitée sont séparées de la mélamine granulaire,
**caractérisé en ce que** l'étape de tamisage grossier est exécutée de sorte que la mélamine granulaire ait une valeur D₉₉ de 300 µm à 1 800 µm.

2. Procédé selon la revendication 1, dans lequel les particules telles qu'elles sont séparées de la mélamine granulaire durant l'étape de tamisage grossier sont renvoyées au moins partiellement à l'étape de broyage et éventuellement transférées au maximum partiellement à la mélamine granulaire provenant de l'étape de tamisage grossier.

3. Procédé selon la revendication 1 ou 2, comprenant en outre une étape de tamisage fin à laquelle la mélamine granulaire produite par l'étape de tamisage grossier est transférée, dans laquelle les particules ayant une taille inférieure à une taille de particule souhaitée sont séparées de la mélamine granulaire, les particules séparées étant ensuite renvoyées partiellement à l'étape de pressage au rouleau et transférées partiellement à la mélamine granulaire provenant de l'étape de tamisage fin.

4. Procédé selon la revendication 3, dans lequel la charge du rouleau est de 0,3 à 7 MN/m.

5. Mélamine granulaire pouvant être obtenue par un procédé selon l'une quelconque des revendications 1-4, **caractérisée en ce que** la valeur D₉₉ est de 300 µm à 1 800 µm.

6. Mélamine granulaire selon la revendication 5, dans laquelle la valeur D₅ est d'au moins 25 µm.

7. Mélamine granulaire selon les revendications 5 ou 6, dans laquelle la mélamine granulaire est obtenue sans utiliser de liant.

8. Mélamine granulaire selon l'une quelconque des revendications 5-7, dans laquelle la mélamine granulaire est obtenue sans étape de comminution avant l'agglomération.

9. Utilisation de mélamine granulaire selon l'une quelconque des revendications 5-8 dans la préparation d'une résine aminoplaste.
